# EUROPEAN PATENT APPLICATION

(11) **EP 1 029 845 A1**
(43) Date of publication of application: **23.08.2000**
(21) Application number: 99301087.5
(22) Date of filing: 15.02.1999
(51) Int. Cl.: C07C 47/11, C07C 45/29, C07C 45/00, C11B 9/00, A61K 7/46

(54) **2-(4-Tert-pentylcyclohexyl)acetaldehyde and its use as fragrance compound**

(71) Applicant: QUEST INTERNATIONAL B.V., 1411 GP Naarden (NL)
(72) Inventor: Munro, David, Maidstone, Kent, ME16 OHF (GB)
(74) Representative: Matthews, Heather Clare

(57) **Abstract**

2-[4-*tert*-pentyl)cyclohexyl]acetaldehyde is a novel compound exhibiting floral, muguet odour characteristics, which finds use in perfumes and in perfumed products.

## Description

### Field of the Invention

This invention concerns a novel fragrance compound, its method of production and use in perfumes and perfumed products.

### Summary of the Invention

In one aspect the invention provides 2-[4-*tert*-pentyl)cyclohexyl]acetaldehyde. This constitutes a novel aldehyde, which has the following structure.

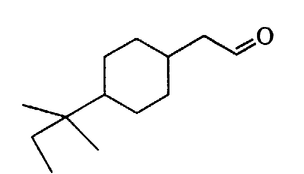

For brevity and simplicity, this material will be referred to herein as "the aldehyde", "the novel aldehyde" or "the aldehyde of the invention".

The aldehyde of the invention exhibits floral, muguet odour characteristics, and so may be used as such to impart, strengthen or improve the odour of a wide variety of products, or may be used as a component of a perfume (or fragrance composition) to contribute its odour character to the overall odour of such perfume. The aldehyde is stable and substantive. For the purposes of this invention a perfume is intended to mean a mixture of fragrance materials, if desired mixed with or dissolved in a suitable solvent or mixed with a solid substrate, which is used to impart a desired odour to the skin and/or product for which an agreeable odour is indispensable or desirable. Examples of such products are: fabric washing powders, washing liquids, fabric softeners and other fabric care products; detergents and household cleaning, scouring and disinfection products; air fresheners, room sprays and pomanders; soaps, bath and shower gels, shampoos, hair conditioners and other personal cleansing products; cosmetics such as creams, ointments, toilet waters, preshave, aftershave, skin and other lotions, talcum powders, body deodorants and antiperspirants, etc.

Other fragrance materials which can be advantageously combined with the aldehyde according to the invention in a perfume are, for example, natural products such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic, and heterocyclic compounds.

Such fragrance materials arc mentioned , for example, in S. Arctander, Perfume and Flavor Chemicals (Montclair. N.J., 1969), in S. Arctander, Perfume and Flavor Materials of Natural Origin (Elizabeth, N.J., 1960) and in "Flavor and Fragrance Materials - 1991", Allured Publishing Co. Wheaton, I11. USA.

Examples of fragrance materials which can be used in combination with the aldehyde according to the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nonpol, nopyl acetate, 2-phenyl-ethanol, 2-penylethyl acetate, benzyl alcohol, benzyl acetate, benzyl salicylate, styrallyl acetate, benzyl benzoate, amyl salicylate, dimethylbenzyl-carbinol, trichloromethylphenyl-carbinyl acetate, p-tert-butylcyclohexyl acetate, isononyl acetate, vetiveryl acetate, vetiverol, α-hexylcinnamaldehyde, 2-methyl-3-(p-tert-butylpheyl)propanal, 2-methyl-3-(p-isopropylphenyl)propanal, 2-(p-tert-butylpheyl)-propanal, 2,4-dimethyl-cyclohex-3-enyl-carboxaldehyde, tricyclodecenyl acetate, tricyclodecenyl propionate,4-(4-hydroxy-4-methylpentyl)-3-cyclohexenecarboxyaldehyde, 4-(4-methyl-3-pentenyl)-3-cyclohexenecarboxaldehyde, 4-acetoxy-3-pentyl-tetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethyl-acetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks, tetralin musks, isochroman musks, macrocyclic ketones, macrolactone musks, ethylene brassylate.

Solvents which can be used for perfumes which contain the aldehyde according to the invention are, for example: ethanol, isopropanol, diethyleneglycol monoethyl ether, dipropylene glycol, diethyl phthalate, triethyl citrate, isopropyl myristate, etc.

The quantities in which the aldehyde according to the invention can be used in perfumes or in products to he perfumed may vary within wide limits and depend, inter alia, on the nature of the product, on the nature and the quantity of the other components of the perfume in which the aldehyde is used and on the olfactive effect desired. It is therefore only possible to specify wide limits, which, however, provide sufficient information for the specialist in the art to be able to use the aldehyde according to the invention for his specific purpose. In perfumes an amount of 0.01% by weight or more of the aldehyde according to the invention will generally have a clearly perceptible olfactive effect. Preferably the amount is 0.1 to 80% by weight, more preferably at least 1 %. The amount of the aldehyde according to the invention present in products will generally be at least 10 ppm by weight, preferably at least 100 ppm, more preferably at least 1000 ppm. However, levels of up to about 20% by weight may be used in particular cases, depending on the product to be perfumed.

The aldehyde exists in *cis* and *trans* forms, both having similar fragrance characteristics, although the *cis*-isomer is more intense, and the invention covers each isomeric form alone, and mixtures of different isomeric forms, and also the use in perfumes and perfumed products of separate isomers and mixtures.

In a further aspect the invention provides a perfume comprising the aldehyde of the invention in an olfactively effective amount.

The invention also covers a perfumed product comprising the aldehyde of the invention.

The aldehyde of the invention may be synthesised in four steps from 4*-tertiary*-amylcyclohexanone. The synthesis route (yields in brackets) is:-
1. Wadsworth-Emmons olefination of 4-*tertiary*-amylcyclohexanone to the unsaturated ester (89%).
2. Catalytic hydrogenation (using Pd/C) to the saturated ester (92%).
3. Lithium aluminium hydride reduction to the cyclohexylethanol (77%).
4. Pyridinium chlorochromate (PCC) oxidation to give 2-[4-*tert*-pentyl)cyclohexyl]acetaldehyde.

In a further aspect of the invention thus provides a method of making 2-[4-*tert*-pentyl)cyclohexyl]acetaldehyde from 4-*tertiary*-amylcyclohexanone, comprising olefination of 4-*tertiary*-amylcyclohexanone to unsaturated ester; catalytic hydrogenation of the unsaturated ester to produce saturated ester; reduction of the saturated ester to produce cyclohexylethanol; and oxidation of cyclohexylethanol to produce 2-[4-*tert*-pentyl)cyclohexyl]acetaldehyde.

The aldehyde of the invention may alternatively by synthesised by performing two sequential Friedel-Crafts alkylations on benzene, the first utilising isoamylene as the alkylating agent to give amylbenzene, and the second using ethylene oxide. This is followed by catalytic reduction to cyclohexyl-2-ethanol and subsequent catalytic dehydrogenation of the side chain to produce the novel aldehyde.

The invention thus also provides a method of making 2-[4-*tert*-pentyl)cyclohexyl] acetaldehyde from benzene, comprising performing two sequential Friedel-Crafts acylations on benzene using isoamylene and ethylene oxide, respectively, as alkylating agents, followed by catalytic reduction to cyclohexyl-2-ethanol and catalytic dehydrogenation of the side chain.

The invention will be further described, by way of illustration, in the following Example and with reference to the accompanying figures in which:

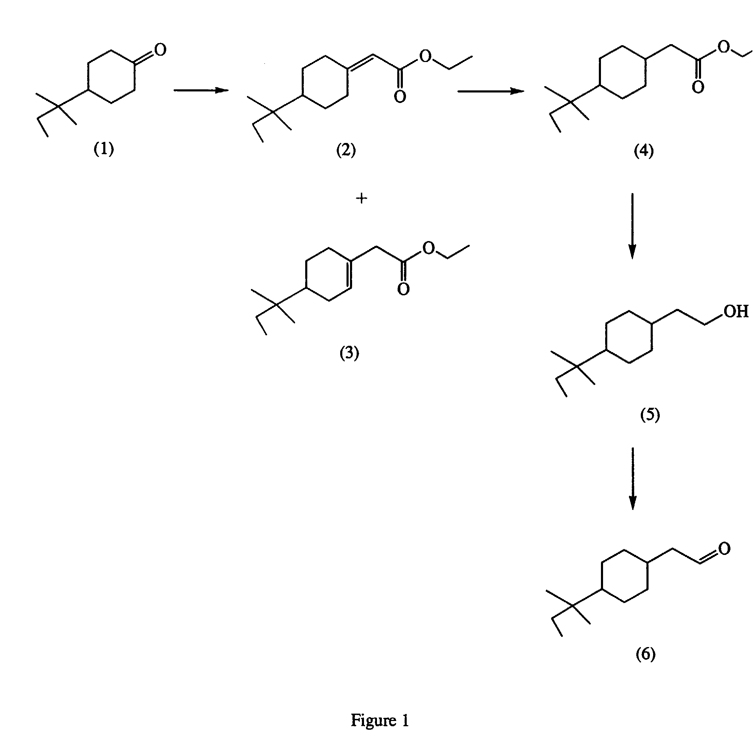

Figure 1 shows a reaction scheme for production of 2-[4-*tert*-pentyl)cyclohexyl]acetaldehyde.

### Example

### Reaction protocol for the synthesis of 2-[4-tert-pentyl)cyclohexyl]acetaldehyde,

The reaction scheme is illustrated in Figure 1.

### A) Synthesis of unsaturated esters by Wadsworth-Emmons reaction

4-*tertiary*-amylcyclohexanone (1 in Figure 1) was converted to a mixture of unsaturated esters, 4-ethoxycarbonylmethylene-*tertiary*-amylcyclohexane and 4-ethoxycarbonylmethyl-*tertiary*-amylcyclohex-3-ene (2 and 3, respectively, in Figure 1).

### Apparatus

A 2 litre 3-necked flask, equipped with a mechanical stirrer, a condenser with calcium chloride guard tube, a thermometer, and a pressure-equalised dropping funnel.

### Method

Triethyl phosphonoacetate (100g, 0.446 mole) was added dropwise, under nitrogen, to a stirred solution of sodium cthoxide freshly prepared by portionwise addition of sodium (10.5g, 0.456 mole) to ethanol (400ml). The temperature rose over the course of the addition (1 hour), from 28°C to 45°C. When the temperature had subsided to 35°C, a solution of 4-*tertiary*-amylcyclohexanone (75g, 0.446 mole) in ethanol (50ml) was added dropwise to the reaction mixture, with stirring under nitrogen, over the course of a further hour. The temperature again rose, from 35°C to 48°C. A 1ml aliquot was taken from the reaction mixture, quenched in a 50/50 mixture of water and diethyl ether (4ml) and checked against the starting materials by TLC (silica stationary phase, chloroform eluent). This indicated the reaction to have gone to completion.

Most ethanol was then removed on a rotary evaporator, and the residue was partitioned between water (400ml) and dichloromethane (500ml). The organic layer was washed with water (400ml), dried over anhydrous MgSO₄, and the solvent removed *in vacuo,* to give a colourless oil which was subjected to flash chromatography (silica stationary phase, chloroform eluent). This gave a colourless oil (94.5g, 89%), consisting of 4-ethoxycarbonylmethylene-*tertiary*-amylcyclohexane (92.2%) and 4-ethoxycarbonylmethyl-*tertiary*-amylcyclohex-3-ene (6.8%). Vacuum distillation using a Vigreux column gave a colourless oil, bp 98°C at 1mbar.

### B) Synthesis of saturated ester by catalytic hydrogenation.

The mixture of unsaturated esters 2 and 3 was converted to the saturated ester 4 in Figure 1.

### Apparatus

1 litre Buchi autoclave

### Method

The mixture of unsaturated esters (2) and (3) (85g, 0.36 mole) from Step A, was dissolved in ethanol (400ml), 5% Pd/C (9g) added, and the reaction mixture charged to the 1 litre Buchi autoclave, where it was stirred under a hydrogen pressure of 6bar for 10 hours, monitoring the reaction by glc [SE52, 100°-200°C at 4°C/min.]. The reaction mixture at this stage consisted of a mixture of *cis-* and *trans-*4-ethoxycarbonylmethyl-*tertiary*-amylcyclohexane (98.8%) (4 in Figure 1).

The catalyst was removed by vacuum filtration through a Celite bed, which was washed thoroughly with ethanol. Removal of solvent *in vacuo* gave a colourless oil, which was purified by flash chromatography (silica stationary phase, chloroform eluent), giving a colourless oil (78.8g, 92%). Vacuum distillation gave a colourless oil, bp 101°C at 1 mbar.

### C) Synthesis of 2-(4-tertiary-amylcyclohexyl)ethanol by lithium aluminium hydride reduction.

### Apparatus

A 2 litre 3-necked flask, equipped with mechanical stirrer, a condenser with calcium chloride guard tube, a thermometer, and a pressure-equalised addition funnel.

### Method

The mixture of *cis-* and *trans*-esters from step B (70.3g, 0.29 mole), dissolved in diethyl in diethyl ether (100mls) was added dropwise under nitrogen to a stirred suspension of lithium aluminium hydride (21g, 0.55 mole) in diethyl ether (500 mls), maintaining the temperature at <30°C by ice-water cooling. After complete addition, ethyl acetate (25mls) was carefully added dropwise, followed by portionwise addition of water (25mls), and, subsequently, 15% NaOH (25mls). The reaction mixture was filtered through Celite, the Celite pad washed copiously with diethyl ether, and the solvent removed *in vacuo* to give a colourless oil (49.3g). TLC (silica stationary phase, 90% chloroform, 10% ethyl acetate eluent), indicated the reaction to have gone to completion. Flash chromatography (conditions as TLC), gave a colourless oil (45.0g, 78%), which consisted of *cis-* and *trans*-2-(4-*tertiary*-amylcyclohexyl)ethanol (5 in Figure 1) (98.8% by glc, conditions as above). A sample was short-path distilled to give a colourless oil, bp 87°C at 0.15mm Hg.

### D) Synthesis of 2-[4-tert-pentyl)cyclohexy]acetaldehyde by PCC oxidation.

### Apparatus

A 2 litre 3-necked flask, equipped with mechanical stirrer, condenser, a thermometer, and a pressure-equalised addition funnel.

### Method

2-(4-*teriary*-amylcyclohexyl)ethanol (58.0g, 0.3 mole) from step C was dissolved and added dropwise under nitrogen to a stirred suspension of pyridinium chlorochromate (PCC) (94.0g, 0.42 mole), maintaining the temperature below 30°C. The reaction mixture was then stirred for a further hour. A 1 ml aliquot was taken, quenched in a mixture of 50/50 water and diethyl ether (4ml), and the ether layer assayed by TLC (silica stationary phase, chloroform eluent), and glc [SE52, 100-200°C at 4°C/min], both of which indicated the reaction to have gone to completion. Diethyl ether (1 litre) was then added to the reaction mixture, which was subsequently separated from the brown, insoluble residue, and passed through a bed of Florisil by vacuum filtration. The solvent was removed *in vacuo* to give a pale yellow oil, which was purified by flash chromatography (conditions as TLC) to give a colourless oil (51.1g). Vacuum distillation through a Vigreux column gave 2-[4-*tert*-pentyl)cyclohexyl]acetaldehyde (also known as 2-[4-*tertiary*-amylcyclohexyl)acetaldehyde) as shown at 6 in Figure 1. Packed column glc (as above) did not separate the *cis-* and *trans-* isomers of the product. The isomers could be seen on capillary glc [25m x 0.2mm, d₁ 3.3 x 10⁻⁷m, SE54, 50-280°C at 3°C/min].

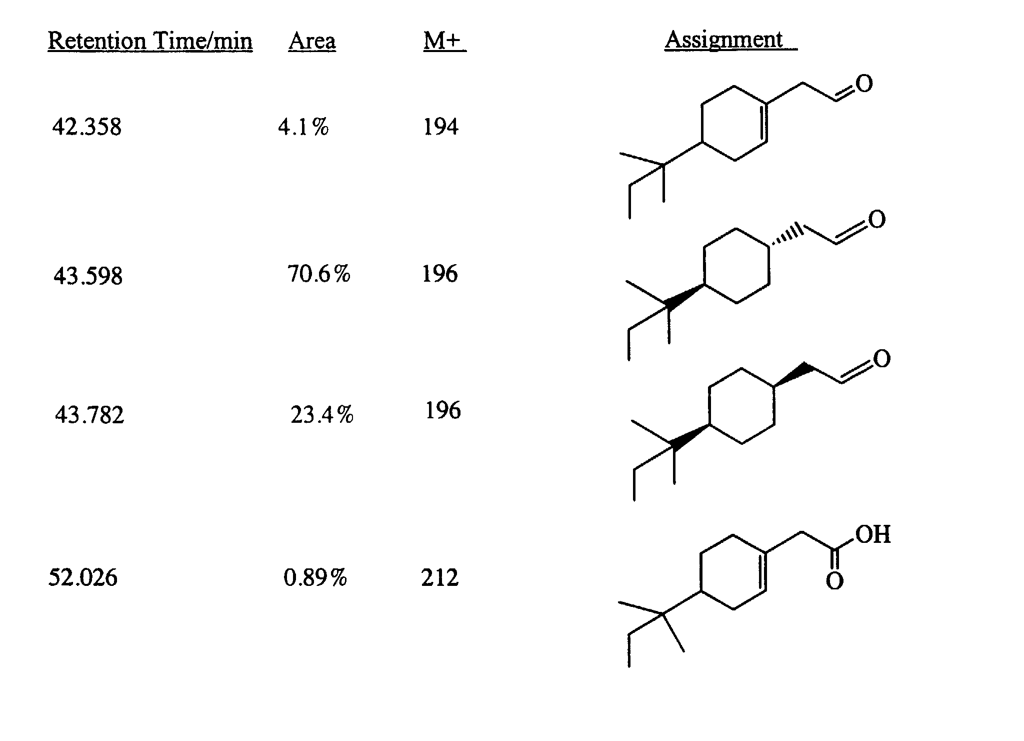

The structural assignment of the product having retention time 42.358 min. is tentative. The carboxylic acid, detected at 52.026 min., is of unknown stereochemistry. However, the stereochemistry of the major products, the *cis-* and *trans*-2-(4-*tertiary*-amylcyclohexyl)acetaldehyde is corroborated by ¹³C NMR, where the minor *cis*-isomer exhibits an upfield chemical shift of the axial methylene. 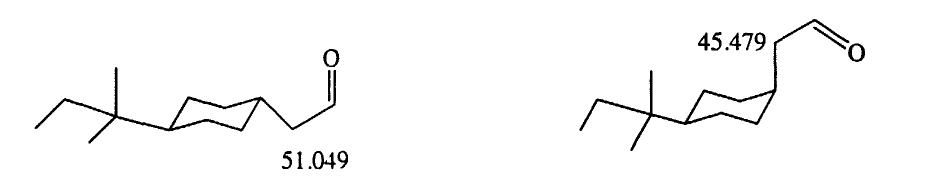 ¹³C NMR (CDCl₃):- 202.264 (CHO), 51.049 (CH₂CHO *trans*-)*,* 45.479 (CH₂CHO *cis*-)*,* 44.625 (CHCH₂CHO), 34.682 (CH₂), 34.179 (CH), 32.853 (CH₂), 32.780 (quaternary), 30.466 (CH₂), 26.726 (CH₂), 23.947 (CH₃ x2, t-amyl), 20.947 (CH₂), 7.835 (CH₃).

The technique of gc-smelling has indicated both isomers to smell strongly of Lilial, the *cis*-isomer being more intense.

The use of flash chromatography as part of the purification process in steps B-D above is optional. It is, however, advisable in step A, in order to remove traces of residual phosphonate. If carried through to the lithium aluminium hydride reduction, stage C, this can give rise to an unpleasant off-note.

On screening, the novel aldehyde 2-[4-*tert*-pentyl)cyclohexyl]acetaldehyde was described as strong, Lilial-like and Bourgeonal-like. Its tenacity on a blotter exceeds three weeks. (Lilial is 3-[4-(*tert*-butyl)phenyl]-2-methylpropanal and Bourgeonal is 3-[4-(*tert*-butyl)phenyl]propanal.)

Stability and performance data so far indicate a cloth substantivity of the novel aldehyde comparable to that of Lilial. The stability of the aldehyde is estimated to be comparable to Lilial.

The novel aldehyde represents a possible replacement for Lilial and its analogues, which are widely used in perfumery for their desirable muguet odours.

## Claims

1. The compound 2-[4-*tert*-pentyl)cyclohexyl]acetaldehyde.

2. The compound of claim 1, comprising *cis* and/or *trans* isomers.

3. A method of making the compound of claim 1 or 2 from 4*-tertiary*-amylcyclohexanone, comprising olefination of 4-*tertiary*-amylcyclohexanone to unsaturated ester; catalytic hydrogenation of the unsaturated ester to produce saturated ester; reduction of the saturated ester to produce cyclohexylethanol; and oxidation of cyclohexylethanol to produce 2-[4-*tert*-pentyl)cyclohexyl]acetaldehyde.

4. A method of making the compound of claim 1 or 2 from benzene, comprising performing two sequential Friedel-Crafts acylations on benzene using isoamylene and ethylene oxide, respectively, as alkylating agents, followed by catalytic reduction to cyclohexyl-2-ethanol and catalytic dehydrogenation of the side chain.

5. A perfume comprising the compound of claim 1 or 2 in an olfactively effective amount.

6. A perfume according to claim 5, wherein the compound is present in an amount of at least 0.01% by weight.

7. A perfume according to claim 6, wherein the compound is present in an amount in the range 0.1 to 80% by weight.

8. A perfumed product comprising a compound according to claim 1 or 2 or a perfume according to claim 5, 6 or 7.
